# FASCICULE DE BREVET EUROPEEN

(11) **EP 0 783 310 B1**
(45) Date de publication et mention de la délivrance du brevet: **09.10.2002**
(21) Numéro de dépôt: 96923018.4
(22) Date de dépôt: 29.07.1996
(51) Int. Cl.: A61K 31/57

(54) **NOUVEAUX MEDICAMENTS HORMONAUX ET LEUR UTILISATION POUR LA CORRECTION DES CARENCES ESTROGENIQUES**
HORMONALE MEDIKAMENTE UND DEREN VERWENDUNG ZUR BEHEBUNG VON ÖSTROGENMANGELERSCHEINUNGEN
NOVEL HORMONAL MEDICAMENTS AND USE THEREOF FOR CORRECTING OESTROGEN DEFICIENCIES

(30) Priorité: 01.08.1995 FR 9509364
(43) Date de publication de la demande: 16.07.1997
(73) Titulaire: LABORATOIRE THERAMEX S.A., 98000 Monaco (MC)
(72) Inventeur: LANQUETIN, Michel, F-06340 La Trinité (FR); PARIS, Jacques, F-06100 Nice (FR); THOMAS, Jean-Louis, F-94220 Charenton-le-Pont (FR)
(74) Mandataire: Burtin, Jean-François
(86) Numéro de dépôt international: IB9600754
(87) Numéro de publication internationale: WO97004784

(56) Documents cités:
- WO-A-94/06437
- DE-A- 3 229 612
- CONTRACEPT. FERTIL. SEX., vol. 22, no. 12, 1994, pages 767-770, XP000568561 D. DE ZIEGLER ET AL.: "The cyclic administration of nomegestrol acetate does not alter the vasodilating effects of estradiol in the uterine artery."
- GYNECOL. REV. GYNECOL., vol. 2, no. 8, 1994, pages 450-454, XP000568560 P. LOPES ET AL.: "Cycle disorders in the perimenopause period."

## Description

La présente invention se rapporte au domaine de la chimie thérapeutique.

Elle a plus précisément pour objet de nouvelles compositions pharmaceutiques formées d'une association estro-progestative en vue de la correction des carences estrogéniques dans les ménopauses naturelles ou artificielles.

Elle a en particulier pour objet une association hormonale, estro-progestative, triséquentielle, caractérisée en ce qu'elle est constituée par des unités de dosage ne renfermant qu'un estrogène, des unités de dosage renfermant une combinaison d'un estrogène et d'un progestatif et des unités de dosage ne contenant qu'un excipient.

Cette association est destinée à être administrée par voie orale et il a été possible, en utilisant une formulation particulière, de pouvoir utiliser par cette voie l'estradiol. Il est possible également d'utiliser un ester d'estradiol ou un produit de conjugaison des estrogènes équins, c'est-à-dire un estrogène naturel.

Comme progestatif on a utilisé un progestatif pur comme l'acétate de nomegestrol. On a, de ce fait, pu constater que dans une telle association l'acétate de nomegestrol n'annihile pas les effets propres de l'estradiol tout en permettant l'obtention d'un cycle artificiel, chez la femme ménopausée, de très bonne qualité.

Elle a spécifiquement pour objet un nouveau médicament estro-progestatif pour la correction des carences estrogéniques caractérisé en ce qu'il est formé de trois types différents d'unités de dosage dont on prévoit l'utilisation selon des séquences successives, à savoir des comprimés de 17β-estradiol, un ester ou un produit de conjugaison, des comprimés contenant à la fois du 17β-estradiol, un ester ou un produit de conjugaison et de l'acétate de nomegestrol et des comprimés placebo ne contenant que l'excipient.

Ces unités d'administration sont destinées a être administrées selon les séquences suivantes :
- les unités de 17β-estradiol, un ester ou un produit de conjugaison pendant dix jours consécutifs
- les unités d'association 17β-estradiol, un ester ou un produit de conjugaison, acétate de nomegestrol pendant quatorze jours consécutifs
- les unités de placebo pendant six jours.

En conséquence, les comprimés de 17β-estradiol, un ester ou un produit de conjugaison seront administrés du jour J1 au jour J10, les comprimés de l'association 17β-estradiol, un ester ou un produit de conjugaison, acétate de nomegestrol seront administrés du jour J11 au jour J24 et les comprimés placebo du jour J25 au jour J30.

Selon un mode d'exécution particulier, les unités de dosage d'estradiol contiennent une quantité de principe actif s'échelonnant de 1 à 3 mg, les unités de dosage contenant l'association de 17β-estradiol, un ester ou un produit de conjugaison et d'acétate de nomegestrol renferment une quantité de 17β-estradiol, un ester ou un produit de conjugaison s'échelonnant de 1 à 3 mg et une quantité d'acétate de nomegestrol s'échelonnant de 1,5 à 6 mg. De préférence, les unités de dosage d'estradiol contiennent de 1 à 2 mg de 17β-estradiol, un ester ou un produit de conjugaison et les unités de dosage de l'association estro-progestative contiennent de 1 à 2 mg de 17β-estradiol, un ester ou un produit de conjugaison et de 2,5 à 5 mg d'acétate de nomegestrol. Spécifiquement les formulations optimales contiennent 1,5 mg de 17β-estradiol, un ester ou un produit de conjugaison ou une association de 1,5 mg de 17β-estradiol, un ester ou un produit de conjugaison et de 3,75 mg d'acétate de nomegestrol.

Ce mode d'administration séquentiel est destiné à compenser les troubles fonctionnels entraînés par une hypoestrogénie liée à la ménopause ou à la pré-ménopause. En particulier, il vise à rétablir un cycle endométrial chez des femmes ménopausées, en particulier celles traitées avec 1 ou 2 mg de 17β-estradiol, un ester ou un produit de conjugaison.

### Première étude clinique :

35 patientes ménopausées ont été étudiées afin de préciser la meilleure dose d'acétate de nomégestrol (**N**), à adjoindre à l'estradiol, pour obtenir des cycles menstruels de qualité avec une bonne imprégnation lutéale de l'endomètre.

Ces patientes ont été suivies dans 6 centres différents et ont reçu : d'abord dans un essai ouvert, pendant 2 mois du 17β-estradiol seul, un ester ou un produit de conjugaison (à raison de 1 ou 2 mg/jour, le gynécologue devant adapter les posologies en fonction de la réponse clinique); ensuite, en aveugle, pendant 4 mois, l'une des associations suivantes (estradiol 10 jours, association les 14 jours suivants, arrêt de 7 jours).
- groupe A : E2 2 mg - N 5 mg (n=6)
- groupe B : E2 2 mg - N 2,5 mg (n=8)
- groupe C : E2 1 mg - N 5 mg (n=9)
- groupe D : E2 1 mg - N 2,5 mg (n=7)
- groupe E : E2 1 mg - N 1,25 mg (n=5)
(E2 : 17β-estradiol N : acétate de nomegestrol)

Dans 6 cas, la posologie de l'estradiol a été adaptée et différait entre les deux phases de traitement.

Deux approches histologiques ont été réalisées : l'une traditionnelle, consistait à différencier de façon semi-quantitative les paramètres de l'estrogénisation et de la lutéinisation endométriale; l'autre consistait à évaluer les même paramètres de façon quantitative à l'aide d'un système informatisé d'analyse d'image.

Au moment de l'étude, les femmes des différents groupes ne différaient par aucun paramètre important (âge, taille, poids, pression artérielle et ancienneté de la ménopause).

Les résultats obtenus après 4 mois de traitement séquentiel de 17β-estradiol/acétate de nomégestrol n'ont pas montré de différence statistiquement significative, d'une façon générale, entre les groupes :
- le plus souvent, pas de réapparition des bouffées de chaleur
- apparition d'une mastodynie plus fréquente dans les deux groupes à 2 mg d'estradiol que dans les trois groupes à 1 mg (respectivement, 4 cas sur 13 et 2 cas sur 18, ces deux derniers dans le groupe E).
- aucune douleur à la palpation des seins, aucun nodule n'ont été observés pendant les six mois de suivi
- survenue d'une hémorragie de privation à la suite de tous les cycles thérapeutiques, excepté dans 1 cas du groupe A et dans 2 cas du groupe C, groupes ayant dans leur composition 5 mg d'acétate de nomégestrol. Le délai d'apparition des hémorragies après l'arrêt de traitement, leur durée et leur abondance, ont été notés.

Sur le plan histologique, il n'y a pas de différence significative entre deux associations estroprogestatives mais elles diffèrent toutes les deux du placebo par l'existence d'un nombre supérieur d'endomètres à l'aspect sécrétoire alors qu'il existe une majorité d'endomètres prolifératifs sous placebo.

En ce qui concerne la survenue de signes cliniques d'hyperestrogénie (mastodynies, douleurs et tension à la palpation des seins, gonflements abdominaux pelviens), il n'y a pas de différence significative entre les trois groupes.
La tolérance générale est équivalente dans les trois groupes. Il n'y a pas de variation de poids ni de la pression artérielle systolique et diastolique après trois mois de traitement.
Dans le groupe placebo, 5 patients présentent un ou plusieurs effets indésirables, 6 dans le groupe 1 mg E2/2,5 mg NOM et 3 dans le groupe 1,5 mg E2/3,75 mg NOM.

En ce qui concerne les paramètres lipidiques, les deux associations progestatives ont entraîné une baisse significative du cholestérol total, du cholestérol LDL et de la Lipoprotéine a (Lpa). Dans le groupe 1,5 mg E2/3,75 mg NOM, on observe une augmentation significative de l'apolipoprotéine A1 et proche de la signification statistique du cholestérol HDL. Il n'y a pas de différence entre les deux associations.
La glycémie et l'insulinémie ne varient pas de façon significative.

En ce qui concerne les facteurs de la coagulation, il n'a pas été observé de modification significative de l'antithrombine III, du fibrinogène, du fragment 1 + 2 de la prothrombine et de la protéine S totale et libre. La protéine C a dominé légèrement dans le groupe 1 mg E2/2,5 mg NOM mais n'a pas varié de façon significative dans le groupe 1,5 mg E2/3,75 mg NOM. La plasminogène a augmenté de façon significative chez les patients traités par les deux associations estroprogestatives.

Ainsi, bien qu'aucune différence statistiquement significative ne puisse être décelée entre les différentes associations, quel que soit le paramètre étudié, clinique ou histologique, les résultats obtenus laissent à penser que le groupe D donne les meilleurs résultats.

### Deuxième étude clinique :

Dans un autre essai, randomisé sur 57 patientes réparties en trois groupes, on a administré en parallèle, dans trois groupes de 19 patientes à raison d'un comprimé par jour :
- des comprimés à 1 mg de 17β-estradiol, un ester ou un produit de conjugaison pendant 24 jours associés à 2,5 mg d'acétate de nomégestrol pendant les 14 derniers jours
- des comprimés à 1,5 mg de 17β-estradiol, un ester ou un produit de conjugaison pendant 24 jours associés à 3,75 mg d'acétate de nomégestrol pendant les 14 derniers jours
- un placebo

A l'inclusion, aucune différence significative entre les 3 groupes n'a été notée en ce qui concerne l'âge, l'ancienneté de la ménopause, l'ancienneté des bouffées de chaleur, les concentrations plasmatiques de FSH et d'estradiol.
Les 3 traitements sont efficaces sur les bouffées de chaleur mais les deux traitements actifs sont différents du placebo en ce qui concerne leur effet sur l'intensité tant au 1er mois, qu'au 3ème mois. En revanche, aucune différence significative entre les deux associations n'a été décelée. En ce qui concerne la fréquence des bouffées de chaleur survenant la nuit, il existe au 1er mois, une différence statistiquement significative entre le groupe 1,5 mg E2/ 3,75 mg NOM et les deux autres.

Le score global de la symptomatologie du climatère diminue de manière significative, que ce soit à 1 ou à 3 mois. Il existe une différence statistiquement significative entre chacune des deux associations estroprogestatives et le placebo à 1 et à 3 mois.

Au niveau de la qualité des cycles, il n'y a pas de différence en ce qui concerne la fréquence de survenue de l'hémorragie de privation, des spotting ou des métrorragies entre les deux groupes traités. Il en est de même pour le délai d'apparition des règles, leur durée et leur abondance.

Sur le plan histologique, il n' y a pas de différence significative entre deux associations estro-progestatives mais elles diffèrent toutes les deux du placebo par l'existence d'un nombre supérieur d'endomètres à l'aspect sécrétoire alors qu'il existe une majorité d'endomètre prolifératif sous placebo.

En ce qui concerne la survenue de signes cliniques d'hyperestrogénie ( mastodynie, douleurs et tension à la palpation des seins, gonflements abdomino-pelviens) il n'y a pas de différence significative entre les trois groupes.
La tolérance générale est équivalente dans les trois groupes. Il n'y a pas de variation du poids ni de la pression artérielle systolique et diastolique après trois mois de traitement;

Dans le groupe placebo, 5 patients présentent un ou plusieurs effets indésirables; 6 dans le groupe 1 mg E2/ 2,5 mg NOM et 3 dans le groupe 1,5 mg E2/ 3,75 mg NOM.

En ce qui concerne les paramètres lipidiques, les deux associations progestatives ont entraîné une baisse significative du cholestérol total, du cholestérol LDL et de la Lpa. Dans le groupe 1,5 mg E2/ 3,75 mg/NOM, on observe une augmentation significative de l'apolipoprotéine A1 et proche de la signification statistique du cholestérol HDL. Il n'y a pas de différence entre les deux associations. La glycémie et l'insulinémie ne varient pas de façon significative.

En ce qui concerne les facteurs de la coagulation, il n'a pas été observé de modification significative de l' antithrombine III, du fibrinogène, du fragment 1 + 2 de la prothrombine et de la protéine S totale et libre. La protéine C a dominé légèrement dans le groupe 1 mg E2/2,5 mg NOM mais n'a pas varié de façon significative dans le groupe 1,5 mg E2/3,75 mg NOM. La plasminogène a augmenté de façon significative chez les patients traités par les deux associations estroprogestatives.

Les concentrations plasmatiques d'estradiol sont plus faibles avec 1 mg d'E2 (35.5 ± 6.67 pg/ml) qu'avec 1,5 mg d'E2 (72.5 ± 6.74 pg/ml). Il existe une différence significative (p < 0,05) dans le groupe 1,5 mg E2/ 3,75 mg NOM par rapport au groupe placebo et par rapport au groupe 1 mg E2/ 2,5 mg NOM ; en ce qui concerne la Sex Hormon Binding Protein : l'augmentation est plus importante dans le groupe plus fortement dosé.

### EN CONCLUSION :

Les deux formulations sont efficaces dès le premier mois sur la symptomatologie du climatère et leur efficacité est différente du placebo. Cependant, en ce qui concerne la fréquence des bouffées de chaleur la nuit, il existe au 1er mois une différence significative entre le groupe « forte dose » et les deux autres groupes.
Aucune différence entre les trois groupes n'a été notée en ce qui concerne la tolérance gynécologique (mastodynie, qualité des cycles et des règles) et générale (poids et pression artérielle).

### Troisième série d'études :

Dans une troisième série d'essais cliniques, on a effectué une étude comparative de l'efficacité thérapeutique de deux associations estroprogestatives contenant des doses différentes de 17β-estradiol et d'acétate de nomégestrol en comparaison avec le placebo, dans le traitement des bouffées de chaleur chez des femmes ménopausées.

Il s'agit d'un essai multicentrique en double aveugle, randomisé en trois groupes parallèles :

83 patientes ont été incluses dans l'étude dont 24 randomisées dans le groupe placebo, 29 dans le groupe 1 mg E2/ 2,5 mg NOM et 30 dans le groupe 1,5 mg E2/3,75 mg NOM portant sur des femmes ménopausées ayant une aménorrhée de plus de 3 mois s'accompagnant de bouffées de chaleur.

Les produits testés ont été :
- comprimés à 1 mg de 17β-estradiol pendant 24 jours avec 2,5 mg d'acétate de nomégestrol dans les 14 derniers comprimés
- comprimés de 1,5 mg de 17β-estradiol pendant 24 jours avec 3,75 mg d'acétate de nomégestrol dans les 14 derniers comprimés
comparativement à un comprimé placebo administré pendant la même durée.

A l'inclusion, l'analyse n'a pas montré de différence entre les trois groupes en ce qui concerne l'âge des patientes, l'ancienneté de la ménopause, le taux de FSH et d'estradiol.

Les résultats de cet essai confirment les données cliniques observées lors des essais précédents en démontrant l'efficacité des deux associations estroprogestatives sur la symptomatologie climatérique et sur les bouffées de chaleur diurnes et nocturnes en particulier. Il n'y a pas de différence entre elles mais elles se révèlent supérieures au placebo sur la plupart des critères cliniques étudiés. Dans les deux groupes sous traitement hormonal, on n'observe pas de différence en ce qui concerne la fréquence de survenue des hémorragies de privation, son débit (d), sa durée et son abondance ni pour les fréquences de survenue des hémorragies intercurrentes (ménorragies et spotting). La tolérance a été équivalente dans les trois groupes étudiés.

### Quatrième étude clinique :

Etude comparative de l'efficacité thérapeutique d'un placebo et de deux associations estroprogestative contenant des doses différentes de 17β-estradiol, un ester ou un produit de conjugaison et d'acétate de nomégestrol sur les marqueurs biologiques du remodelage osseux chez des femmes ménopausées.

La méthodologie est celle d'un essai multicentrique en double aveugle randomisé sur 3 groupes parallèles.

Le nombre de sujets est de 117 (38 dans le groupe placebo, 39 dans le groupe 1 mg E2/ 2,5 mg NOM et 40 dans le groupe 1,5 mg E2/ 3,75 mg NOM)
Il s'agit de femmes ménopausées ayant une aménorrhée de plus de 6 mois.

PRODUIT, DOSE et MODE D'ADMINISTRATION :
- comprimés à 1 mg de 17β-estradiol, un ester ou un produit de conjugaison pendant 24 jours avec 2,5 mg d'acétate de nomégestrol dans les 14 derniers comprimés
- comprimés à 1,5 mg de 17β-estradiol, un ester ou un produit de conjugaison pendant 24 jours avec 3,75 mg d'acétate de nomégestrol dans les 14 derniers comprimés
- comprimés placebo

### Résultats :

Les données cliniques recueillies dans cet essai confirment les résultats des essais précédents en montrant que les deux associations diminuent la fréquence des bouffées de chaleur et permettent le rétablissement d'un cycle artificiel.

Aucune différence significative n'a été trouvée entre les trois groupes en ce qui concerne la tolérance clinique et biologique.

Les deux associations estroprogestatives testées se différencient du placebo par leur aptitude à faire baisser de façon significative les phosphatases alcalines sanguines et les rapports pyridinoline/créatinine et désoxypyridinoline/créatinine dans les urines alors que ces paramètres augmentent dans le groupe placebo.

Ces résultats obtenus à court terme indiquent donc que les associations hormonales testées sont à même de freiner le remodelage osseux augmenté après la ménopause. Ceci laisse présager un effet bénéfique dans la prévention de l'ostéoporose postménopausique.

L'analyse intragroupe des marqueurs plasmatiques du remodelage osseux montre une diminution significative de l'ostéocalcine (p < 0,02) et proche de la significativité des phosphatases alcalines dans le groupe 1 mg E2/ 2,5 mg NOM.

La comparaison du groupe placebo et des deux groupes traités met en évidence une différence significative (p < 0,05) en ce qui concerne les phosphatases alcalines et proche de la significativité (p=0,065) en ce qui concerne leurs isoenzymes osseux. Il n'y a pas de différence entre les deux groupes traités.

Pour ce qui est des marqueurs urinaires, il n'y a pas de différence entre les 3 groupes au niveau des rapports calcium/créatinine et hydroxyproline/créatinine. Au niveau du rapport pyridinoline/créatinine, il existe une différence significative entre le groupe 1 mg E2/ 2,5 mg NOM et le groupe placebo quand on compare le pourcentage de variation par rapport à la valeur d'inclusion.

Le score global de la symptomatologie du climatére diminue significativement dans les trois groupes sans différence entre eux.

Il n'existe pas de différence entre les deux groupes traités en ce qui concerne la fréquence de survenue des hémorragies de privation, des spotting et des métrorragies. Cependant, deux patientes ont arrêté le traitement pour métrorragies dans le groupe à 1,5 mg E2/ 3,75 mg NOM, aucune dans les deux autres groupes. En ce qui concerne la durée des règles, il n'y a pas de différence entre les deux groupes traités alors que le délai d'apparition est plus court dans le groupe à 1 mg E2/ 2,5 mg NOM que dans le groupe à dose forte (p < 0,05).

En ce qui concerne les mastodynies, les gonflements abdomino-pelviens, les douleurs et la tension à la palpation des seins, bien que leur survenue ait tendance à être plus fréquente dans le groupe 1,5 mg E2/ 3,75 mg NOM, la différence avec les deux autres groupes n'est pas significative.

La tolérance est équivalente dans les trois groupes de même que l'incidence du nombre d'arrêts de traitement. Un ou plusieurs effets secondaires ont été rencontrés chez 5 patients du groupe placebo, 13 du groupe 1 mg E2/ 2,5 mg NOM, 12 du groupe 1,5 mg E2/ 3,75 mg NOM.
Les pressions artérielles systoliques et diastoliques ne sont pas modifiées, quel que soit le traitement. Il existe une augmentation significative du poids (p < 0,01) dans le groupe 1,5 mg E2/ 3,75 mg NOM au cours de l'essai; cependant, il n'y a pas de différence significative entre les trois groupes tant au niveau du poids que de la pression artérielle.

Concernant les paramètres biologiques, il existe une augmentation proche de la significativité de la glycémie dans le groupe 1 mg E2/ 2,5 mg NOM et une diminution proche de la significativité du cholestérol dans le groupe placebo. La comparaison des paramètres biologiques métaboliques n'a pas montré de différence significative entre les trois groupes.
Les concentrations plasmatiques d'estradiol sont plus faibles avec 1 mg d'E2 (49.6 ± 8.09 pg/ml) qu'avec 1,5 mg d'E2 (60.8 ± 10.24 pg/ml).

### EN CONCLUSION :

En ce qui concerne la plupart des marqueurs du remodelage osseux, il n'y a pas de différence entre les deux groupes traités et le groupe placebo, à l'exception du pourcentage de variation du rapport pyridinoline/créatinine par rapport à l'inclusion; il existe une différence entre le groupe placebo et le groupe « faible dose ».

### Exemple de composition pharmaceutique selon l'invention :

### A/. Comprimés d'estradiol :

| | |
|---|---|
| Estradiol | 1,500 g |
| Polyvinylpyrrolidone | 13,500 g |
| (Kollidon 25 de la Société BASF) | |
| Lactose | 135,795 g |
| Cellulose microcristalline | 26,250 g |
| (Avicel PH 101) | |
| Palmitostearate de glyceryle | 2,775 g |
| (Precirol) | |
| Silice colloïdale anhydre | 1,000 g |
| (Aerosil 200) | |
| Crospovidone | 4,000 g |
| (Polyplasdone XL) | |
| Agent de coloration pour 1.000 comprimés terminés à 0,185 g. | 0,180 g |

La production se fait en deux temps :

| **a) préparation d'un pré-mélange** | |
|---|---|
| Estradiol hemi-hydrate (avec une hydratation moyenne de 3,2 %) | 0,4644 |
| Polyvinylpyrrolidone | 4,050 kg |
| Alcool isopropylique | 3,532 kg |
| Eau purifiée | 2,025 kg |
| Lactose | 18,000 kg |
| Cellulose microcristalline | 2,250 kg |
| Total après granulation et séchage | # 24,764 kg |

| **b) Préparation du mélange final :** | |
|---|---|
| Pré-mélange granulé et séché | # 24,7640 kg |
| Palmitostearate de glyceryle | 0,8325 kg |
| Silice colloïdale anhydre | 0,300 kg |
| Polyvinyl pyrrolidone reticulée | 1,200 kg |
| Cellulose microcristalline | 5,6250 kg |
| Lactose | 22,7385 kg |
| Agent colorant | 0,0540 kg |
| | 55,5000 kg |

### B/. Comprimés d'Estradiol et d'acétate de Nomegestrol (1,5 mg Estradiol et 3,75 mg d'acetate de nomegestrol par comprimé) :

| | |
|---|---|
| Acetate de nomegestrol | 0,3750 kg |
| Pré-mélange selon Aa) | 8,9285 kg |
| Lactose | 6,4000 kg |
| Avicel PH 101 | 1,8010 kg |
| Precirol ATO 5 | 0,2775 kg |
| Polyplasdone XL | 0,6000 kg |
| Agent colorant | 0,0180 kg |
| Aerosil 200 | 0,1000 kg |
| pour 100.000 comprimés terminés au poids moyen de 0,185 g. | |

## Revendications

1. Association hormonale estroprogestative triséquentielle **caractérisée en ce qu'**elle est constituée par 10 unités journalières de dosage ne renfermant qu'un estrogène, par 14 unités journalières de dosage renfermant une combinaison d'un estrogène et d'un progestatif et par 6 unités journalières de dosage ne contenant qu'un excipient.

2. Association hormonale selon la revendication 1 dans laquelle l'estrogène est le 17β-estradiol, un ester de 17β-estradiol ou un produit de conjugaison de l'estradiol.

3. Association hormonale selon la revendication 1 dans laquelle le progestatif est l'acétate de nomegestrol.

4. Association hormonale selon l'une des revendications 1 ou 2 dans laquelle l'estrogène seul est administré sous la forme de comprimés de 17β-estradiol, d'un ester de 17β-estradiol ou d'un produit de conjugaison de l'estradiol.

5. Association hormonale estroprogestative pour le traitement des carences estrogéniques et le rétablissement d'un cycle artificiel chez la femme ménopausée, par voie orale, selon la revendication 2 dans laquelle le 17β-estradiol, l'ester de 17β-estradiol ou le produit de conjugaison de l'estradiol, est présent à une dose allant de 1 à 3 mg par prise unitaire.

6. Association hormonale estroprogestative selon la revendication 5 dans laquelle le 17β-estradiol, l'ester de 17β-estradiol ou le produit de conjugaison de l'estradiol est présent dans la composition estrogène à une dose allant de 1 à 2 mg et de préférence à une dose de 1,5 mg.

7. Association hormonale estroprogestative utilisée pour le traitement des carences estrogéniques et le rétablissement d'un cycle artificiel chez la femme ménopausée par voie orale, selon la revendication 1, **caractérisée en ce que** dans l'unité de dosage renfermant la combinaison d'un estrogène et d'un progestatif, l'estrogène est présent à une dose allant de 1 à 3 mg, et l'acétate de nomegestrol est présent à une dose allant de 1,5 à 6 mg par prise unitaire.

8. Association hormonale estroprogestative selon la revendication 7 dans laquelle le 17β-estradiol, l'ester ou le produit de conjugaison de l'estradiol est présent à une dose allant de 1 à 2 mg, et l'acétate de nomegestrol à une dose allant de 2,5 à 5 mg.

9. Procédé de préparation d'une association estroprogestative selon la revendication 5 **caractérisé en ce que**:
a) la composition estrogénique est préparée par une méthode qui consiste à préparer d'abord un pré-mélange composé d'estradiol hemi-hydrate, de polyvinylpyrrolidone, d'alcool isopropylique, d'eau purifiée, de lactose et de cellulose microcristalline, puis, après granulation et séchage, à préparer le mélange final composé du pré-mélange granulé et séché, de palmito-stéarate de glycéryle, de silice colloïdale anhydre, de polyvinyl pyrrolidone réticulée, de cellulose microcristalline, de lactose et d'agent colorant, et
b) la composition estro-progestative est préparée pour une méthode qui consiste à additionner le pré-mélange défini an point a) d'acétate de Nomegestrol, d'excipients classiques pharmaceutiques, d'un agent colorant et de silice colloïdale pour former des comprimés contenant du 17β-estradiol et de l'acétate de Nomegestrol.

## Patentansprüche

1. Hormonale trisequentielle Östrogen/Progestativum-Assoziation, **dadurch gekennzeichnet, dass** sie aus folgenden Einheiten besteht: 10 tägliche Dosierungseinheiten, die nur ein Östrogen enthalten, 14 tägliche Dosierungseinheiten, die eine Kombination aus einem Östrogen und einem Progestativum enthalten, und 6 tägliche Dosierungseinheiten, die nur eine Grundmasse enthalten.

2. Hormonale Assoziation nach Anspruch 1, **dadurch gekennzeichnet, dass** das Östrogen das 17β-Östradiol, ein 17β-Östradiolester oder ein konjugiertes Östradiol ist.

3. Hormonale Assoziation nach Anspruch 1, **dadurch gekennzeichnet, dass** das Progestativum das Nomegestrolacetat ist.

4. Hormonale Assoziation nach Anspruch 1 oder 2, **dadurch gekennzeichnet, dass** das alleinige Östrogen in Form von Tabletten verabreicht wird, die aus 17β-Östradiol, einem 17β-Östradiolester oder einem konjugierten Östradiol bestehen.

5. Hormonale Östrogen/Progestativum-Assoziation zur Behandlung von Östrogenmangelerscheinungen und zur Wiederherstellung eines künstlichen Zyklus bei postmenopausalen Frauen, die oral verabreicht wird, nach Anspruch 2, **dadurch gekennzeichnet, dass** das 17β-Östradiol, der 17β-Östradiolester oder das konjugierte Östradiol in einer Dosis von 1 bis 3 mg pro Einnahmeeinheit vorliegt.

6. Hormonale Assoziation nach Anspruch 5, **dadurch gekennzeichnet, dass** das 17β-Östradiol, der 17β-Östradiolester oder das konjugierte Östradiol in der Östrogen-Zusammensetzung in einer Dosis von 1 bis 2 mg, vorzugsweise in einer Dosis von 1,5 mg, vorliegt.

7. Hormonale Östrogen/Progestativum-Assoziation zur Behandlung von Östrogenmangelerscheinungen und zur Wiederherstellung eines künstlichen Zyklus bei postmenopausalen Frauen, welche oral verabreicht wird, nach Anspruch 1, **dadurch gekennzeichnet, dass** in der die Kombination eines Östrogens und eines Progestativums enthaltenden Dosierungseinheit das Östrogen in einer Dosis von 1 bis 3 mg und das Nomegestrolacetat in einer Dosis von 1,5 bis 6 mg pro Einnahmeeinheit vorliegen.

8. Hormonale Assoziation nach Anspruch 7, **dadurch gekennzeichnet, dass** das 17β-Östradiol, der Ester oder das konjugierte Östradiol in einer Dosis von 1 bis 2 mg und das Nomegestrolacetat in einer Dosis von 2,5 bis 5 mg pro Einnahmeeinheit vorliegen.

9. Verfahren zur Herstellung einer Östrogen/Progestativum-Assoziation nach Anspruch 5, **dadurch gekennzeichnet, dass**
a) die Östrogen-Zusammensetzung nach einem Verfahren hergestellt wird, das darin besteht, zunächst eine Vormischung vorzubereiten, die aus Halbhydratöstradiol, Polyvinylpyrrolidon, Isopropylalkohol, gereinigtem Wasser, Lactose und mikrokristalliner Cellulose besteht und dann, nach der Granulierung und Trocknung, die Endmischung herzustellen, die aus der granulierten und getrockneten Vormischung, aus Glyzeryl-Palmito-Stearat, wasserfreier Kolloid-Kieselsäure, vernetztem Polyvinylpyrrolidon, mikrokristalliner Cellulose, Lactose und Farbstoffen besteht, und
b) die Östrogen-Progestativum-Zusammensetzung nach einem Verfahren hergestellt wird, die darin besteht, zur Bildung von 17β-Östradiol und Nomegestrolacetat enthaltenden Tabletten der unter Punkt a) definierten Vormischung Nomegestrolacetat, klassische pharmazeutische Grundmasse, einen Farbstoff und Kolloid-Kieselsäure beizugeben.

## Claims

1. Hormonal, estroprogestative trisequential combination **characterized in that** it consists of 10 daily dosage units only containing an estrogen, of 14 daily dosage units containing a combination of an oestrogen and a progestative agent and of 6 daily dosage units merely containing an excipient.

2. Hormonal combination according to claim 1, wherein the oestrogen is 17β-estradiol, an ester of 17β-estradiol or a conjugation product of estradiol.

3. Hormonal combination according to claim 1, wherein the progestative agent is Nomegestrol acetate.

4. Hormonal combination according to one of claims 1 or 2, wherein the estrogen alone is given in the form of tablets of 17β-estradiol or of an ester of 17β-estradiol or of a conjugation product of estradiol.

5. Estroprogestative hormonal combination for the treatment of estrogenic deficiencies and the restoration of an artificial cyclus in the menopaused women according to claim 2, wherein the 17β-estradiol, the ester of estradiol or the conjugation product of estradiol is present at a dosis ranging from 1 to 3 mg per unit dosage.

6. Estroprogestative hormonal combination according to claim 5, wherein 17β-estradiol, the ester of 17β-estradiol or the conjugation product of estradiol is present in the estrogenic composition at a dosis ranging from 1 to 2 mg and preferably at a dosis of 1,5 mg.

7. Estroprogestative hormonal combination intended for the treatment of estrogenic deficiencies and the restoration of an artificial cyclus in the menopaused women, per oral way, according to claim 1, **characterized in that** in the unit dosage containing the combination of an estrogen and a progestative agent the estrogen is present at a dosis ranging from 1 to 3 mg and Nomegestrol acetate is present at a dosis ranging from 1,5 to 6 mg per unit dosage.

8. Estroprogestative hormonal combination according to claim 7, wherein 17β-estradiol, the ester or the conjugation product of estradiol is present at a dosis ranging from 1 to 2 mg, and Nomegestrol acetate at a dosis ranging from 2,5 to 5 mg.

9. Process for the preparation of an estroprogestative combination according to claim 5, **characterized in that**:
a) the estrogenic composition is prepared using a method which consists first preparing a premix made of estradiol hemihydrate, polyvinylpyrrolidone, isopropyl alcohol, purified water, lactose, microcrystalline cellulose, then after granulation and drying, in preparing the final mixture made of granulated and dried premix, of glyceryl palmito-stearate, anhydrous colloidal silica, cross-linked polyvinylpyrrolidone, microcrystalline cellulose, lactose and colouring matter and
b) preparing the estroprogestative composition using a method which consists in adding the premix defined at point a) with Nomegestrol acetate, usual pharmaceutical diluents, a colouring agent and colloidal silica to produce tablets containing 17β-estradiol and Nomegestrol acetate.
